# EUROPEAN PATENT APPLICATION

(11) **EP 4 349 486 A1**
(43) Date of publication of application: **10.04.2024**
(21) Application number: 22199375.1
(22) Date of filing: 03.10.2022
(51) Int. Cl.: B01L 3/00, C12M 3/06, C12N 5/00

(54) **MICROFLUIDIC DEVICE WITH A MULTIPLE-COATED SURFACE COMPRISING AT LEAST TWO DIFFERENT POLYPEPTIDES**

(71) Applicant: LUMICKS CA Holding B.V., 1105 AG Amsterdam (NL)
(72) Inventor: CHOCKLEY, Peter James, AMSTERDAM (NL); GREGG, Trillian Ashley, AMSTERDAM (NL); BAILEY, Keith Alan, 1105 AG Amsterdam (NL)
(74) Representative: Algemeen Octrooi- en Merkenbureau B.V.

(57) **Abstract**

The invention relates to devices for measuring cellular avidity and to processes for making and using the devices.

## Description

### Field of the invention

The invention relates to devices for measuring cellular avidity and to processes for making and using the devices.

### Background of the invention

Microfluidics is an area of research that addresses and exploits the fluid dynamics at millimeter-to-submillimeter scale. The emerging and rapid development of microfluidic technology has presented an ideal solution for the problem of mimicking an *in vivo* like microenvironment. This has led to microfluidics based setups being developed for a large range of applications in almost all scientific disciplines including chemistry, biochemistry and biophysics.

Microfluidics have also been used in cell biology applications. One of the benefits of microfluidic devices is the possibility of integrating analytical methods to produce information from the cell models.

An analytical method that has started to gain much interest in recent years is cell avidity. Cell avidity evaluates the overall strength of cellular interactions by defining the total intercellular force between multiple parallel interactions, including co-receptor binding, TCR clustering, cell adhesion proteins, and even orientations and valencies. Cell avidity therefore provides a complete and physiologically relevant picture reflecting the interaction between cells, for example target cells and effector cells.

At the heart of the cell avidity technology is a microfluidic device comprising a monolayer of target cells. Robust and confluent cell monolayers are crucial to be able to do reliable and accurate cell avidity experiments.

It is therefore desirable to provide new and innovative concepts, designs and processes aimed at preparing robust and confluent cell monolayers in microfluidic devices.

### Detailed description of the invention

One skilled in the art will readily appreciate that the present invention is well adapted to carry out the objects and obtain the ends and advantages mentioned as well as those inherent therein. It should be understood, however, that the materials, compounds, coatings, processes, procedures, and techniques described herein are presently representative of preferred embodiments. These techniques are intended to be exemplary, are given by way of illustration only, and are not intended as limitations on the scope. Other objects, features, and advantages of the present invention will be readily apparent to one skilled in the art from the detailed description, specific examples and claims; and various changes, substitutions, other uses and modifications that may be made to the invention disclosed herein without departing from the scope and spirit of the invention or as defined by the scope of the appended claims.

As used herein other than the claims, the terms "a," "an," "the," and "said" means one or more. As used herein in the claim(s), when used in conjunction with the words "comprises" or "comprising," the words "a," "an," "the," or "said" may mean one or more than one. As used herein "another" may mean at least a second or more.

The present application describes a process for preparing a microfluidic device comprising a multiple-coated surface, the process comprising the steps of:
- providing a microfluidic device comprising a surface,
- providing a first coating comprising at least a polypeptide,
- coating the surface with the first coating to provide a microfluidic device comprising a single-coated surface,
- providing a second coating comprising at least a polypeptide, and
- coating the single-coated surface with the second coating to provide a microfluidic device comprising a multiple-coated surface.

The present application also describes a process for preparing a microfluidic device comprising a multiple-coated surface, the process comprising the steps of:
- providing a microfluidic device comprising a surface coated with a first coating comprising at least a polypeptide,
- providing a second coating comprising at least a polypeptide, and
- coating the surface coated with the first coating with the second coating to provide a microfluidic device comprising a multiple-coated surface.

The present application also describes a process for preparing a microfluidic device comprising a surface comprising cells, the process comprising the steps of:
- performing a process for preparing a microfluidic device comprising a multiple-coated surface as described herein,
- providing cells, and
- contacting the cells with the multiple-coated surface and allowing the cells to attach to the multiple-coated surface to provide a microfluidic device comprising a surface comprising cells.

In an embodiment the surface coated with a first coating comprising at least a polypeptide is activated before the first coating is applied. In an embodiment the surface is chemically activated. Activation can be done to facilitate application of the first coating.

The process for preparing a microfluidic device comprising a multiple-coated surface can be a process comprising the steps as described above.

A surface on which a coating has been applied is known herein as a "coated surface." The term "single-coated surface" as used herein means a surface that comprises at least a coating layer comprising at least a polypeptide. In an embodiment the single-coated surface comprises at least the first coating as described herein. The term "multiple-coated surface" as used herein means a surface that comprises at least two coating layers comprising at least a polypeptide. In an embodiment the multiple-coated surface comprises a first coating layer comprising at least a polypeptide and at least a second coating layer comprising at least a polypeptide. The multiple-coated surface may comprise additional coating layers. Said additional coating layers may be below the first coating layer (i.e. between the surface and the first coating layer), between the first coating layer and the second coating layer and/or on top of the second coating layer. In an embodiment the surface may be coated multiple times with the first coating layer and/or multiple times with the second coating layer. In other words, the coating process can be repeated numerous times until the desired order of coating layers is achieved. For example, the surface may be coated in the following order: first coating, second coating, first coating, second coating or, alternatively, first coating, first coating, second coating. In a preferred embodiment the surface does not comprise any additional coating layers next to the first coating layer and the second coating layer.

In an embodiment the polypeptide comprised in the first coating is different from the polypeptide comprised in the second coating. In an embodiment the first coating comprises more than one polypeptide. In an embodiment the second coating comprises more than one polypeptide. In an embodiment the first coating comprises more than one polypeptide and the second coating also comprises more than one polypeptide. In case a coating comprises more than one polypeptide, e.g. two, three, four, etc polypeptides, said polypeptides may differ and/or may be present in the coating in a suitable ratio.

In an embodiment the cells form a monolayer on the surface. In an embodiment the surface is a glass surface, a plastic surface, a metal surface, a ceramic surface or any combination thereof. The microfluidic device may be prepared from any suitable material including plastic, glass, ceramics, metal or any combination thereof. Preferably, the plastic is non-cytotoxic. The plastic may include natural polymers and/or synthetic polymers.

In an embodiment the cell monolayer shows a high quality. Quality can be measured for example as shown in the examples by looking at the confluency of the cell monolayer. Preferably, the confluency should be 50% or higher, preferably 60% or higher, more preferably 70% or higher. Quality can also be measured for example as shown in the examples by looking at the clumpiness of the cell monolayer. Preferably, the cell monolayer does not show clumpiness. Quality can also be measured for example by looking at the stability and/or robustness of the cell monolayer. Stability and/or robustness can be measured for example as shown in the examples by measuring the resistance of the cell monolayer when subjected to shear flow force and/or acoustic force.

In an embodiment the cells are cultured. Culturing of the cells may take place before, simultaneously with and/or after contacting the cells with the multiple-coated surface and allowing the cells to attach to the multiple-coated surface to provide a microfluidic device comprising a surface comprising cells.

In an embodiment the polypeptide comprised in the first coating is selected from the group consisting of poly-L-lysine, poly-D-lysine, poly-L-ornithine, concanavalin A, and active domains thereof, and any combination thereof. In an embodiment the first coating comprises at least two polypeptides. In an embodiment the first coating comprises poly-L-lysine and concanavalin A.

In an embodiment the polypeptide comprised in the second coating is selected from the group consisting of laminin, collagen, fibronectin, fibrinogen, vitronectin, osteopontin, procollagen, elastin, tenascin, entactin, thrombospondin, osteocalcin, von Willibrand Factor, cadherins, protocadherins, desmogleins, desmocollins, and active domains thereof, and any combination thereof.

In an embodiment the second polypeptide is fibrinogen.

In an embodiment the second polypeptide is a cadherin. In an embodiment the cadherin is selected from the group consisting of E-cadherin (CDH1), N-cadherin (CDH2), N-cadherin 2 (CDH12), P-cadherin (CDH3), R-cadherin (CDH4), VE-cadherin (CDH5), K-cadherin (CDH6), cadherin 7 (CDH7), cadherin 8 (CDH8), cadherin 9 (CDH9), cadherin 10 (CDH10), OB-cadherin (CDH11), T-cadherin (CDH13), M-cadherin (CDH15), KSP-cadherin (CDH16), LI-cadherin (CDH17), cadherin 18 (CDH18), cadherin 19 (CDH19), cadherin 20 (CDH20), cadherin 23 (CDH23), CDH22, CDH24, CDH26, CDH28, CELSR1, CELSR2, CELSR3, CLSTN1, CLSTN2, CLSTN3, DCHS1, DCHS2, LOC389118, PCLKC, RESDA1, RET, and active domains thereof, and any combination thereof. In an embodiment the cadherin is E-cadherin. In an embodiment the desmoglein is selected from the group consisting of DSG1, DSG2, DSG3, DSG4, and active domains thereof, and any combination thereof. In an embodiment the desmocollin is selected from the group consisting of DSC1, DSC2, DSC3, and active domains thereof, and any combination thereof. In an embodiment the protocadherin is selected from the group consisting of PCDH1, PCDH7, PCDH8, PCDH9, PCDH10, PCDH11X/11Y, PCDH12, PCDH15, PCDH17, PCDH18, PCDH19, PCDH20, PCDHA1, PCDHA2, PCDHA3, PCDHA4, PCDHA5, PCDHA6, PCDHA7, PCDHA8, PCDHA9, PCDHA10, PCDHA11, PCDHA12, PCDHA13, PCDHAC1, PCDHAC2, PCDHB1, PCDHB2, PCDHB3, PCDHB4, PCDHB5, PCDHB6, PCDHB7, PCDHB8, PCDHB9, PCDHB10, PCDHB11, PCDHB12, PCDHB13, PCDHB14, PCDHB15, PCDHB16, PCDHB17, PCDHB18, PCDHGA1, PCDHGA2, PCDHGA3, PCDHGA4, PCDHGA5, PCDHGA6, PCDHGA7, PCDHGA8, PCDHGA9, PCDHGA10, PCDHGA11, PCDHGA12, PCDHGB1, PCDHGB2, PCDHGB3, PCDHGB4, PCDHGB5, PCDHGB6, PCDHGB7, PCDHGC3, PCDHGC4, PCDHGC5, FAT, FAT2, FAT4, and active domains thereof, and any combination thereof.

An "active domain" refers to an amino acid sequence found within the polypeptide that, in itself, provides function according to one or more properties of the polypeptide, such as providing structural support to cells and/or for attaching cells. The peptide that includes an active domain of a polypeptide can have a "core sequence" of amino acid residues, and optionally one or more additional amino acid residues that flank (i.e., on the C-terminus, N-terminus, or both) the core sequence. The one or more additional amino acids that flank the core sequence can correspond to the wild-type polypeptide sequence in the relevant region of the protein, or can comprise one or more amino acid(s) that diverge from the wild-type sequence (e.g., a "variant amino acid sequence"). The variant amino sequence can be one that enhances properties of the peptide, such as providing enhanced ligand interaction, and/or can facilitate formation of the second coated layer. As used herein, a "peptide" is a short polymer of 25 or less amino acids linked by peptide bonds. As used herein, a "polypeptide" is a polymer of more than 25 amino acids linked by peptide bonds and which includes full length proteins. A peptide having an active portion of a polypeptide can be synthesized by solid phase peptide synthesis (SPPS) techniques using standard techniques, such as Fmoc synthesis.

In an embodiment the first coating layer comprising the polypeptide(s) may comprise other excipients and/or components as well. In an embodiment the polypeptide is present in the first coating layer in an amount of at least 10% (w/w) (weight percentage of the polypeptide based on the total weight of the first coating layer comprising the polypeptide), at least 20% (w/w), at least 30% (w/w), at least 40% (w/w), at least 50% (w/w), at least 60% (w/w), at least 70% (w/w), at least 80% (w/w), at least 90% (w/w) or at least 100% (w/w).

In an embodiment the second coating layer comprising the polypeptide(s) may comprise other excipients and/or components as well. In an embodiment the polypeptide is present in the second coating layer in an amount of at least 10% (w/w) (weight percentage of the polypeptide based on the total weight of the second coating layer comprising the polypeptide), at least 20% (w/w), at least 30% (w/w), at least 40% (w/w), at least 50% (w/w), at least 60% (w/w), at least 70% (w/w), at least 80% (w/w), at least 90% (w/w) or at least 100% (w/w).

In an embodiment the polypeptide is present in the first coating layer in an amount of 1 pg/ml - 5 mg/ml.

In an embodiment the polypeptide is present in the second coating layer in an amount of 1 pg/ml - 5 mg/ml.

The first and second coating can be prepared to have a desired thickness. In an embodiment, the coated layers have a thickness in the range of about 5 nm to about 50 nm.

The first and second coating can be prepared to have a desired density and/or concentration.

In an embodiment the coating process involves placing the coating materials (e.g. polypeptides of the first and/or second coating) in contact with the device surface. In an embodiment the device surface has been pretreated with a base coat. In some modes of practice, the coating materials are applied to a surface and dried down or partially dried down. The process of applying can be performed using any one of a variety of techniques including dipping, pouring, swabbing, siphoning, brushing, rolling, padding, ragging, painting, spraying, anodizing, electroplating, and/or laminating. One exemplary method for applying a coating composition is by dip coating. A typical dip coating procedure involves immersing the surface to be coated in a first coating composition, dwelling the object in the composition for a period of time, and then removing the surface from the composition. After the surface has been dip coated in the coating solution, it is removed and dried or partially dried. Drying can be carried out using any suitable method, including air drying the surface.

In an embodiment the processes as described herein also comprise the step of contacting the microfluidic device comprising a surface comprising cells with a blocking agent. The blocking agent may reduce background binding to the cells. Examples of suitable blocking agents include, but are not limited to, N-hydroxysuccinimide (NHS) ester reagents. An NHS ester reagent as defined herein is a compound comprising at least one succinimidyl group, which functional group is capable of forming an ester bond with a primary amine group on polypeptides, such as, for example, the amine groups as comprised in lysine residues. such that these amine groups are no longer available to attach to, for example, cells of interest.

Preferably, the NHS ester reagent comprises a spacer, e.g. a polymer of a relative short length, up to about 100 Angstrom. Such a polymer preferably is inert. Preferably, the polymer is polyethylene glycol (PEG). More preferably, the PEG length is up to 25 ethylene glycol units in length. Preferably, the number of units is selected from the range of 2 to 25, or from 3 to 10. Hence, in a further embodiment, the NHS ester is a PEG-NHS ester.

Blocking may take place by providing a buffer suitable for the blocking reaction and sustaining cell viability of the cells present on the surface, e.g. phosphate, carbonate-bicarbonate, HEPES or borate buffers at pH 7.2 to 8.5 for 0.5 to 4 h at room temperature or 4°C. Once the blocking reaction has been allowed to take place for a defined period, the NHS ester reagent is removed and the cells may, for example, be washed with serum containing medium or with a primary amine buffer such as Tris or glycine, which are not compatible because they compete for reaction, to quench (stop) the reaction. NHS esters are known in the art, and it may be contemplated to utilize other compounds equally capable of reacting with amine groups as an alternative. These compounds include, but are not limited to, isothiocyanates, isocyanates, acyl azides, sulfonyl chlorides, aldehydes, glyoxals, epoxides, oxiranes, carbonates, aryl halides, imidoesters, carbodiimides, anhydrides, and fluorophenyl esters. Most of these compounds conjugate to amines by either acylation or alkylation. Without being bound by theory, as long as the compound is capable of reacting with the polypeptide responsible for the coating on the surface, it can be contemplated.

In an embodiment the cells (i.e. the cells that are contacted with the multiple-coated surface) are target cells or effector cells. In a preferred embodiment the cells are target cells. In an embodiment the target cells are cells presenting an antigen. For example, a tumor antigen, a viral antigen or a bacterial antigen. Suitable target cells may include eukaryotic cells or prokaryotic cells. Target cells that may be selected include fungal cells, animal cells, insect cells, mammalian cells, bacterial cells, yeasts, and protozoa. In a preferred embodiment the target cells are human cells. In a preferred embodiment the target cells are tumor cells such as human tumor cells. In an embodiment the effector cells are human effector cells such as T cells, NK cells, B cells, dendritic cells, macrophages, or monocytes. The effector cells may be genetically modified. When the genetic modification is with a CAR, cells such as CAR T cells can be obtained. Effector cells as used herein may thus also be CAR T cells.

In an embodiment extraneous DNA is removed from the cells before contacting the cells with the multiple-coated surface. In an embodiment the extraneous DNA is removed from the cells by treatment of the cells with a deoxyribonuclease (DNAse).

The present application also describes a process for determining cellular avidity comprising:
- providing a microfluidic device comprising a surface comprising cells, wherein the surface comprises a first coating comprising at least a polypeptide, a second coating comprising at least a polypeptide and cells attached to the second coating,
- providing cells of interest,
- incubating the cells attached to the second coating with the cells of interest, and
- determining the cellular avidity of the cells of interest with the cells attached to the second coating.

The term "cellular avidity" as used herein is defined by the overall strength of interactions occurring in a cell-to-cell contact, involving a diversity of molecules at the surfaces of the cells that interact. *Ergo,* cellular avidity is a parameter that expresses the strength of binding between cells. It involves a multitude of interactions, including a diversity of receptor-ligand pairs, among which e.g. a specific receptor-ligand interaction, occurring at the membrane surface of a cell, working jointly forming a strong bond between cells. It may also involve active signalling and processes internal to the cells such as e.g. during immune synapse formation. It is understood that the cellular avidity of a cell of a certain type is defined relative to its target cell and conditions tested. It is understood that in accordance with the invention target cells and cells of interest relate to two different cells which are to interact specifically with each other. For example, the target cells or the cells of interest may express a ligand on their surface and the other cell may express a receptor for that ligand. As control cells, the same cells as the cells of interest or target cells are utilized, but these cells do not express such a ligand or receptor or express a variant thereof which is not functional. As said, with regard to the cells attached to the second coating (e.g. target cells) and cells of interest (e.g. effector cells) (and control cells thereof), it is understood that this may involve cells that are to have a specific interaction, i.e. one cell carrying a receptor and the other cell having a ligand for the receptor. The term ligand and receptor in this sense and in accordance with the invention may define their inter-relationship. The term receptor may not be construed to be limiting in any way and is understood to mean a protein presented at the cell surface which can (specifically) interact with another protein (ligand) presented at a another cell. The terms ligand and receptor are used to indicate a complementarity which is important for specific recognition between cells without restrictions on the complementary molecules that can be contemplated. Receptors that may be of interest include, but are not limited to, a CAR, a TCR, a stimulatory or inhibitory coreceptor, or a receptor engaged via a bispecific antibody.

Advantageously, of a variety of cells of interest the cellular avidity may be determined. This is for example of interest when it is desirable to provide for a candidate CAR. By providing a variety of CARs, candidates can be selected and subsequently assessed with regard to functionality, e.g. by *in vivo* experiments. As cellular avidity is an important parameter for function, this way highly efficiently, suitable candidates can be selected. Accordingly, in a further embodiment, of a variety of cells of interest the cellular avidity is determined.

In an embodiment the microfluidic device comprising a surface comprising cells is prepared by performing a process for preparing a microfluidic device comprising a surface comprising cells as described herein.

In an embodiment the cells are target cells or effector cells. In an embodiment the cells of interest are target cells or effector cells. In a preferred embodiment the cells attached to the second coating are target cells and the cells of interest are effector cells. In an embodiment the target cells are cells presenting an antigen. For example, a tumor antigen, a viral antigen or a bacterial antigen. Suitable target cells may include eukaryotic cells or prokaryotic cells. Target cells that may be selected include fungal cells, animal cells, insect cells, mammalian cells, bacterial cells, yeasts, and protozoa. In a preferred embodiment the target cells are human cells. In a preferred embodiment the target cells are tumor cells such as human tumor cells. In an embodiment the effector cells are human effector cells such as T cells, NK cells, B cells, dendritic cells, macrophages, or monocytes. The effector cells may be genetically modified. When the genetic modification is with a CAR, cells such as CAR T cells can be obtained. Effector cells as used herein may thus also be CAR T cells.

The cells that are attached to the second coating are preferably attached as a monolayer. The subsequent cells of interest (and control cells thereof) that are to interact with the cells attached to the coating on the surface are preferably provided in a relatively low cell density as compared with those cells, such that substantially all cells of interest (and control cells thereof) can interact with those cells. In other words, there are more cells per cell of interest. Such provides for advantageous controllable conditions when applying the force on the cells of interest or the control cells thereof.

In an embodiment control cells of the target cells or the cells of interest are provided and the cellular avidity of the control cells is determined as well. In an embodiment control cells of the cells that are attached to the second coating and/or control cells of the cells of interest are provided and the cellular avidity of the control cells is determined as well. The process for determining cellular avidity as described herein may be performed separately with cells of interest and their control cells and/or with cells that are attached to the second coating and their control cells. When the control cells are of the cells of interest, the process for determining cellular avidity as described herein may comprise the step of incubating the cells attached to the second coating with the cells of interest and/or their control cells. In other words, the process for determining cellular avidity as described herein may comprise the step of contacting cells of interest and/or their control cells with target cells. Control cells and/or cells of interest that have detached and/or remained attached are determined and cellular avidity scores are provided for the control cells and/or cells of interest with the cells that are attached to the second coating. Instead of providing control cells of the cells of interest, alternatively control cells of the cells that are attached to the second coating may be provided attached to a surface. In such a scenario, cells of interest are contacted with the cells that are attached to the second coating and before or thereafter cells of interest are contacted with the control cells of the cells that are attached to the second coating, and in each case cells of interest that have detached and/or remained attached are determined and cellular avidity scores are provided for the cells of interest with the control cells of the cells that are attached to the second coating and with the cells that are attached to the second coating as such. The cellular avidity scores obtained for cells and their respective controls can be compared and are of interest.

One may also perform the process for determining cellular avidity as described herein in a combined fashion, e.g. when cells of interest and their control cells are differentially labelled or can be otherwise distinguished from each other when determining cellular avidity. It is understood that in accordance with the invention cells that are attached to the second coating and cells of interest relate to two different cells which are to interact specifically with each other, e.g. one cell expressing a ligand on its surface, whereas the other cell expresses a receptor for that ligand.

In an embodiment, when cells of interest are used that express a specific ligand or receptor, control cells are used that are the same cells as the cells of interest but do not express the specific ligand or receptor or express a variant of the specific ligand or receptor which is not functional or not specific for the counterpart present on the cells that are attached to the second coating, e.g. the target cells. Obviously, the same is true *vice versa.*

In general, as long as a force can be applied to the cells binding to the cells attached to the second coating, and detachment and/or attachment of the cells can be determined, determination of the cell numbers thereof allows one to determine cellular avidity.

In an embodiment the cellular avidity is determined by exerting a force on the cells of interest. As long as a force can be applied and controlled on cells of interest that bind/interact/attach with cells attached to a surface, e.g. target cells, such a force can be contemplated in accordance with the invention. Preferably, the force is an acoustic force, a shear flow force or a centrifugal force.

In an embodiment cellular avidity is measured using the z-Movi^{®} device as available from the company Lumicks. The device makes use of an acoustic force. A microfluidic device as described herein can be used in the z-Movi^{®} device. It may even be used repeatedly.

With regard to cellular avidity, it is to be understood that this is to express the strength of binding of cells of interest (or control cells thereof) to cells attached to a surface (or control cells thereof). It is to be understood that where it is referred to specific forces applied to cells, this may refer to average forces, e.g. such forces may not be fully homogeneous, for example over the contact surface.

In a preferred embodiment, the cellular binding avidity is determined by exerting a force on the cells of interest, e.g. effector cells, away from the cells that are attached to the second coating, e.g. target cells. It is understood that in this step, the force applied may be perpendicular (in the direction of z-axis) to the surface (x,y) to which the cells that are attached to the second coating are attached, for example when a centrifugal force or acoustic force is applied. The force may also be lateral (x-axis or y-axis), for example when a shear force is applied. In any case, the force is applied and is controlled such that a defined force is exerted on the cells of interest that interact with the cells that are attached to the second coating. It is understood that the force that is exerted on the cells of interest attached to the cells that are attached to the second coating is to be substantially equal. This can be achieved when using for example a flat surface. Other suitable surface shapes may be used (e.g. a tube with exerted concentrical force or laminar flow force in the direction of the length of the tube), as long as the force exerted can be substantially equal at a defined surface area, such a surface shape may be contemplated. The force required to move a cell of interest away from the cells that are attached to the second coating (i.e. a cell detachment event) can be detected optically, e.g. via microscopy or other means. Cell detachment events can be monitored and counted.

In an embodiment the process for determining cellular avidity may further comprise the step of collection of cells, for example, collection of cells that experienced a defined force. In an embodiment the cells of interest may be provided with a photoactivatable label which may subsequently be activated by illumination with light of a suitable wavelength only in a well-defined interaction region of the device (e.g. in a center region under an (acoustic) force transducer) to photoactivate and/or switch on the dye. Subsequently, the cells can be sorted, for example, using fluorescence activated cell sorting (FACS) and only those cells which are activated are collected thereby obtaining the cells on which defined forces have been exerted. This may for example be highly useful for collecting cells that remained attached to the cells on the surface, e.g. the target cells. For example, the target cells and cells of interest bound thereto may be trypsinized thereby obtaining both the target cells and cells of interest that remained bound thereto in a suspension. Alternatively, attached cells of interest can also be simply collected with physical means (e.g. by scraping) from the area of interest, i.e. the surface area with a well-defined nominal force.

It is to be understood that with regard to the force exerted, the exact forces experienced by cells may also depend on cell size and or other cell properties such as density and compressibility. The force may thus be a nominal force and not the true force experienced by the cells. Since it may be hard to precisely predict the average cell size, density, compressibility, etc. of the cells, the force may have been calculated based on theory alone or may have been calibrated using test particles with specific properties (see Kamsma, D. et al. (2016). Tuning the Music: Acoustic Force Spectroscopy (AFS) 2.0. Methods, 105, 26-33). The force may be a calculated or calibrated force expressed with units of N (e.g. pN), but it may also be expressed without calibration as the input power (Vpp) applied to, for example, a piezo element (see Sitters, G. et al. (2014). Acoustic force spectroscopy. Nature Methods, 12(1), 47-50), as angular velocity squared (*ω*²) in the case of centrifugal forces or as flow speed v and or as shear stress (Pa) in case of shear forces. As long as the forces exerted by the devices can be varied and controlled and reproduced in such devices, such devices are suitable for use in the processes for determining cellular avidity as described herein.

As the percentage of cells of interest that remains bound at a certain applied force is indicative of cellular avidity, i.e. the larger the percentage of cells of interest that is bound, the higher the cellular avidity, it is useful to refer to the percentage of cells. Of course, a different measure which relates to cellular avidity may be used, for example the percentage of detached cells, wherein conversely a low number is indicative of a higher cellular avidity. Instead of percentage, the ratio of cells that remain attached or are detached divided by the total number of cells that interacted may be provided. In case of a cellular avidity plot, the area under (or above) the curve may be determined. In case a fixed number of cells of interest is to be provided to a surface comprising cells, simply provide the number of cells of interest that have detached and/or remained attached. In any case, as long as a unit is provided that is representative of the number of cells of interest that have detached or cells of interest that have remained attached, relative to the total number of cells that have interacted, such a unit may be contemplated. Such a unit allows for ranking cellular avidities, when comparing e.g. different cells of interest. Providing such a unit may be referred to as providing a cellular avidity score. A preferred cellular avidity score may be the percentage of cells of interest that remains attached relative to the cells of interest that have interacted, the latter being set at 100%. Hence, in determining cellular avidity a cellular avidity score is preferably provided.

Any suitable force application method may be contemplated in processes for determining cellular avidity as described herein. Preferably, increasing the force is well controlled. In a further embodiment, the applied force is a force ramp, preferably a linear force ramp. It is understood that when a force is selected to be applied it can be a constant force applied for a defined period. The forces applied may be in various forms as a function of time. Preferably however, the applied force is an increasing force. That is, after the incubation step, an increasing force is applied for a defined period until a defined end force is reached. For example, a linear force ramp may be applied for 150 seconds resulting in a defined end force of 1000 pN.

In a further embodiment, the ratio or difference between the cellular avidity of cells of interest and control cells is determined. The ratio of the cellular avidity between cells of interest and control cells is preferably as large as possible. Ideally, control cell background binding is about 15% or less and cells of interest binding is about 85% or more, for example from 85-95%. In an embodiment the percentage difference between cellular avidity of control cells and cells of interest is at least 30%, at least 40%, at least 50%, at least 60% at least 70%, at least 80% and preferably at least 90%. Preferably, conditions are selected with the largest difference between the percentages. In an embodiment wherein e.g. conditions are to be selected for sorting purposes and the like, it may be desirable to have no control cells remaining attached or at least a low percentage, such as 5% or less, 4% or less, or 3% or less. With any other unit or cellular avidity score determined, optimal differences can likewise be selected.

In an embodiment, the present application also describes processes for identifying a candidate agent capable of modulating the cellular avidity between a cell of interest and a cell that is attached to the second coating, wherein in the incubation step, and cellular avidity determination step of the processes as described herein agent(s) are provided and included in these steps. This way, when it is for instance of importance to block or enhance a particular interaction, agent(s) capable of doing so can be identified. Furthermore, the cells that are attached to the second coating can also be used in processes for screening of different cells of interest, by performing the processes as described herein, optionally in the presence of agent(s) as defined above, and comparing determined cellular avidities for the different cells of interest. This way, for example, in case agents are present that are known to modulate a desired interaction between a cell of interest and a target cell, receptor/ligand interactions may be selected that are not affected by such agents, or, conversely, are aided or even strengthened by such agents. Hence, in one embodiment, a candidate agent is provided for modulating the avidity between a cell of interest and a cell that is attached to the second coating, and wherein said agent is present in the incubation step and step of determining cellular avidity.

In an embodiment the processes for determining cellular avidity as described herein may comprise the step of providing a cell engager. Cell engagers include antibodies, or the like, which are capable of binding to a target cell and a cell of interest, e.g. an effector cell. Such antibodies may include single chain antibodies comprising two binding domains such as scFv domains, and include BiTEs (i.e. bispecific T-cell engagers) or the like. A conventional antibody design includes heavy and light chains, with one half of the antibody (one heavy chain and one light chain) engaging with a target cell, and the other half of the antibody (another heavy chain and another light chain) engaging with an effector cell, wherein preferably, the Fc domain is made inert. In any case, suitable cell engagers are widely known in the art and the current invention allows to study and/or determine cellular avidity induced by a cell engager between a target cell and an effector cell. Hence, a cell engager may be provided in addition and the cellular avidity score may be determined, induced by said cell engager, between a cell of interest (e.g. an effector cell) and a target cell. In an embodiment said cell engager has a binding region capable of binding the cell of interest (e.g. an effector cell) and a binding region capable of binding the target cell. It is understood that the incubation step in the processes as described herein can thus be performed in the presence of a cell engager to allow the cells, i.e. effector cells and target cells, to interact and form a bond via the cell engager. Accordingly, in an embodiment, in processes as described herein, a cell engager is provided capable of binding an effector cell and a target cell, and the cell engager is included in the incubation step. Furthermore, such processes are highly useful for screening cell engagers, e.g. by identifying cell engagers that are particular capable of binding an effector cell and a target cell.

Of course, in case cells that are attached to the second coating as prepared in accordance with the invention are provided, with an optional modulating agent, and shown to provide for selectivity with regard to binding of cells of interest to these cells, as opposed to control cells, such can also be used for selecting and/or sorting cells of interest. Hence, in another embodiment, processes are provided for selecting and/or sorting cells of interest, comprising the steps of the processes as described herein, and optionally in the presence of agent(s) as defined above, comprising the further step of applying the selected force on the cells of interest and subsequently selecting and/or sorting cells of interest that have detached and/or that remain attached to the cells that are attached to the second coating. Hence, in another embodiment, a process is provided comprising the steps of the processes of determining cellular avidity as described herein, wherein the process is for use in sorting and/or screening of cells of interest.

Accordingly, a process is provided for sorting and/or screening of cells of interest comprising the steps of:
- providing a microfluidic device comprising a surface comprising cells, wherein the surface comprises a first coating comprising at least a polypeptide, a second coating comprising at least a polypeptide and cells attached to the second coating,
- providing cells of interest,
- incubating the cells attached to the second coating with the cells of interest,
- applying a force on the cells of interest, and
- selecting and/or sorting cells of interest that have detached and/or that remain attached to the cells attached to the second coating.

In an embodiment the microfluidic device comprising a surface comprising cells is prepared by performing a process for preparing a microfluidic device comprising a surface comprising cells as described herein.

In a further embodiment, processes may be of use for the screening of candidate agents as well. Hence, a further process is provided for screening candidate agents for modulating cellular avidity between of cells of interest and cells attached to the second coating comprising the steps of:
- providing a microfluidic device comprising a surface comprising cells, wherein the surface comprises a first coating comprising at least a polypeptide, a second coating comprising at least a polypeptide and cells attached to the second coating,
- providing cells of interest,
- incubating the cells attached to the second coating with the cells of interest,
- applying a force on the cells of interest, and
- determining cells of interest that have detached and/or that remain attached to the cells attached to the second coating and provide a cellular avidity score.

In an embodiment the microfluidic device comprising a surface comprising cells is prepared by performing a process for preparing a microfluidic device comprising a surface comprising cells as described herein.

Cells of interest which can be selected and/or sorted and thus be obtained accordingly with processes as described herein may subsequently in a further step be admixed with a pharmaceutically acceptable buffer or otherwise pharmaceutical acceptably formulated.

The present application also describes a microfluidic device obtainable by a process for preparing a microfluidic device comprising a surface comprising cells as described herein.

The present application also describes a microfluidic device obtained by a process for preparing a microfluidic device comprising a surface comprising cells as described herein.

The present application also describes a microfluidic device comprising a surface, wherein the surface comprises a first coating comprising at least a polypeptide and a second coating comprising at least a polypeptide.

The present application also describes a microfluidic device comprising a surface, wherein the surface comprises a first coating comprising at least a polypeptide, a second coating comprising at least a polypeptide, and cells attached to the second coating.

In an embodiment a microfluidic device as described herein comprises one or more internal spaces including a vessel, typically in the form of a flow channel. The flow channel may be configured to convey a flow of fluid. The microfluidic device as described herein may also comprise one or more compartments configured to contain one or more fluids. The microfluidic device as described herein may also comprise one or more connectors for connecting purposes. In an embodiment the microfluidic device comprises a unitary body in which the one or more internal spaces are at least partly formed.

The present application also describes a cell culture device comprising a surface, wherein the surface comprises a first coating comprising at least a polypeptide and a second coating comprising at least a polypeptide. A "cell culture device" as used herein means a receptacle that can contain media for culturing a cell or tissue. The cell culture device may include glass, ceramics, metal, plastic or any combination thereof. Preferably, the plastic is non-cytotoxic. The plastic may include natural polymers and/or synthetic polymers. Exemplary cell culture devices include, but are not limited to, single and multi-well plates, including 6-well and 12-well culture plates, and smaller welled culture plates such as 96-, 384-, and 1536-well plates, culture jars, culture dishes, petri dishes, culture flasks, culture plates, culture roller bottles, culture slides, including chambered and multi-chambered culture slides, culture tubes, coverslips, cups, spinner bottles, perfusion chambers, bioreactors, and fermenters.

A cell culture device as described herein may also be used in a process for determining cellular avidity as described herein. A cell culture device as described herein may also be used in a process for sorting or screening cells of interest as described herein. A cell culture device as described herein may also be used in a process for screening candidate agents for modulating cellular avidity between cells as described herein.

In an embodiment of the microfluidic devices as described herein the polypeptide comprised in the first coating is selected from the group consisting of poly-L-lysine, poly-D-lysine, poly-L-ornithine, concanavalin A, and active domains thereof, and any combination thereof.

In an embodiment of the microfluidic devices as described herein the polypeptide comprised in the second coating is selected from the group consisting of laminin, collagen, fibronectin, fibrinogen, vitronectin, osteopontin, procollagen, elastin, tenascin, entactin, thrombospondin, osteocalcin, von Willibrand Factor, cadherins, protocadherins, desmogleins, desmocollins, and active domains thereof, and any combination thereof. In an embodiment the polypeptide comprised in the second coating is a fibrinogen. In an embodiment the polypeptide comprised in the second coating is a cadherin. In an embodiment the cadherin is selected from the group consisting of E-cadherin (CDH1), N-cadherin (CDH2), N-cadherin 2 (CDH12), P-cadherin (CDH3), R-cadherin (CDH4), VE-cadherin (CDH5), K-cadherin (CDH6), cadherin 7 (CDH7), cadherin 8 (CDH8), cadherin 9 (CDH9), cadherin 10 (CDH10), OB-cadherin (CDH11), T-cadherin (CDH13), M-cadherin (CDH15), KSP-cadherin (CDH16), LI-cadherin (CDH17), cadherin 18 (CDH18), cadherin 19 (CDH19), cadherin 20 (CDH20), cadherin 23 (CDH23), CDH22, CDH24, CDH26, CDH28, CELSR1, CELSR2, CELSR3, CLSTN1, CLSTN2, CLSTN3, DCHS1, DCHS2, LOC389118, PCLKC, RESDA1, RET, and active domains thereof, and any combination thereof. In an embodiment the cadherin is E-cadherin. In an embodiment the desmoglein is selected from the group consisting of DSG1, DSG2, DSG3, DSG4, and active domains thereof, and any combination thereof. In an embodiment the desmocollin is selected from the group consisting of DSC1, DSC2, DSC3, and active domains thereof, and any combination thereof. In an embodiment the protocadherin is selected from the group consisting of PCDH1, PCDH7, PCDH8, PCDH9, PCDH10, PCDH11X/11Y, PCDH12, PCDH15, PCDH17, PCDH18, PCDH19, PCDH20, PCDHA1, PCDHA2, PCDHA3, PCDHA4, PCDHA5, PCDHA6, PCDHA7, PCDHA8, PCDHA9, PCDHA10, PCDHA11, PCDHA12, PCDHA13, PCDHAC1, PCDHAC2, PCDHB1, PCDHB2, PCDHB3, PCDHB4, PCDHB5, PCDHB6, PCDHB7, PCDHB8, PCDHB9, PCDHB10, PCDHB11, PCDHB12, PCDHB13, PCDHB14, PCDHB15, PCDHB16, PCDHB17, PCDHB18, PCDHGA1, PCDHGA2, PCDHGA3, PCDHGA4, PCDHGA5, PCDHGA6, PCDHGA7, PCDHGA8, PCDHGA9, PCDHGA10, PCDHGA11, PCDHGA12, PCDHGB1, PCDHGB2, PCDHGB3, PCDHGB4, PCDHGB5, PCDHGB6, PCDHGB7, PCDHGC3, PCDHGC4, PCDHGC5, FAT, FAT2, FAT4, and active domains thereof, and any combination thereof.

The microfluidic devices and/or cell culture devices as described herein can be used for cell avidity measurements and for sorting of cells of interest, they however may also be used to conduct one or more of a wide range of investigations of interactions between cells and one or more substances. Such interactions to be investigated may include, but are not limited to, an effect of the substance(s) on one or more of: cell attachment, cell growth, cell survival, cell differentiation, cell apoptosis/death and multicellular structure. The activity or interaction of substances on cell motility, cell migration, cell to cell interactions as well as cell-protein signal interactions are also important activities that can be studied using the microfluidic devices and/or cell culture devices as described herein. Substances of interest in such investigations may include, but are not limited to, pharmaceutical compounds or other therapeutics, exosomes, nanomicelles, nanoparticles, toxins, small molecules, nucleic acids (including nucleic acid vectors), oligonucleotides (including antisense oligonucleotides), oligopeptides, proteins, ribozymes, small interfering RNAs, microRNAs, short hairpin RNAs, aptamers, viruses, and antibodies or antigen binding parts thereof. These examples are non-limiting and other substances are contemplated as well.

The present application also describes a kit of parts comprising a microfluidic device comprising a surface, a first coating comprising at least a polypeptide, and a second coating comprising at least a polypeptide.

The present application also describes a kit of parts comprising a microfluidic device comprising a surface comprising a first coating comprising at least a polypeptide, and a second coating comprising at least a polypeptide.

A microfluidic device present in the kit of parts refers to a device having the required structural configuration of a microfluidic devices as described herein, but in which a first coating comprising at least a polypeptide and/or a second coating comprising at least a polypeptide is yet to be formed.

The constituents of the kits of parts as described herein may be stored in one or more containers prior to their application and/or use. The kits of parts as described herein may be multi-pack kits wherein different components are stored in a plurality of containers.

A kit of parts as described herein may further comprise instructions for coating the surface with the first and/or second coating and, optionally, instructions for contacting and attaching cells with a surface coated with the second coating to provide a microfluidic device comprising a surface comprising cells.

Any of the features and embodiments according to the processes as described herein also apply to the microfluidic devices and kits as described herein.

### Examples

### Example 1

### Monolayer quality of single-coated and multiple-coated flow cells

The microfluidic devices used in the experiment were z-Movi^{®} chips (obtained and as available from LUMICKS). The chips were coated with:
a) poly-L-Lysine (Sigma) diluted 1:5 in Dulbecco's phosphate buffered saline (DPBS) for 10 minutes at room temperature. Thereafter the chips were dried for 5 minutes using air, or
b) poly-L-Lysine (Sigma) diluted 1:5 in Dulbecco's phosphate buffered saline (DPBS) for 10 minutes at room temperature. Thereafter, the chips were dried for 5 minutes using air. Next, the chips were coated using 2.5 µg/ml recombinant human E-cadherin in DPBS for 60 minutes at 37°C.

MCF7 cells were grown in culture in RPMI medium comprising 20% (v/v) fetal bovine serum (FBS) and 1% (w/v) glutamine for 24-48 hours at 37°C and 5% CO₂ until 70-90% confluency was reached. The cells were then treated for 5 minutes at 37°C with 1 ml undiluted TrypLE^{™} Express (Thermo Fisher). Next, the TrypLE^{™} Express was blocked by using at least 4x the volume (compared to the TrypLE^{™} Express volume used) of RPMI medium comprising 20% (v/v) fetal bovine serum (FBS) and 1% (w/v) glutamine and the cells were spun down at 200xg. The cells were resuspended in 1 ml of RPMI medium comprising 20% (v/v) fetal bovine serum (FBS), 1% (w/v) glutamine and 10 U/ml DNase I and incubated for 5 minutes. Next, the cells were spun down at 200xg and resuspended in RPMI medium comprising 20% (v/v) fetal bovine serum (FBS) and 1% (w/v) glutamine at a concentration of 80-100e6 cells/ml.

The chips were loaded with 12.5 µl of the cell suspension using pipetting. After 45 minutes the medium was refreshed and 75 minutes later the chips were ready for use.

Next, the cell monolayer quality was analysed by analysing four parameters: the confluency of the cell monolayer, the clumpiness of the cell monolayer, the resistance of the cell monolayer to shear flow force and the resistance of the cell monolayer to acoustic force.

The four parameters were measured by visually checking the monolayer in the z-Movi^{®} chip. The chip is placed on the z-Movi^{®} cell avidity analyzer (obtained and as available from LUMICKS) and the monolayer was observed using the software of the z-Movi^{®} cell avidity analyzer,

The z-Movi^{®} cell avidity analyzer contains a microscope with a 10x magnification lens. The confluency of the monolayer was visually analysed.

By scrolling through different focusses and visual analysis, it was determined if there is clumping of cells. If there is clumping, different layers of cells are observed. If there is no clumping, one cell layer is observed.

The resistance of the monolayer to shear flow force was checked by creating negative pressure in the chip, flowing cell culture medium through the chip and observing the cell monolayer in parallel. There is resistance to shear flow force, if the cells stay stuck in the monolayer. When cells are released, there is no resistance to shear flow force.

Lastly, an acoustic force of 1000pN was applied by the z-Movi^{®} cell avidity analyzer. There is resistance to acoustic force, if cells stay bound. When cells are released, there is no resistance to acoustic force.

The results are shown in Table 1.

The results show that when the flow cells are multiple-coated the obtained cell monolayer has a much higher confluency, does not show clumpiness of cells and has resistance against shear flow force and acoustic force compared to flow cells that have been single-coated.

### Example 2

### Monolayer quality of single-coated and multiple-coated flow cells

The microfluidic devices used in the experiment were z-Movi^{®} chips (obtained and as available from LUMICKS). The chips were coated with:
a) poly-L-Lysine (Sigma) diluted 1:5 in Dulbecco's phosphate buffered saline (DPBS) for 10 minutes at room temperature. Thereafter the chips were dried for 5 minutes using air, or
b) poly-L-Lysine (Sigma) diluted 1:5 in Dulbecco's phosphate buffered saline (DPBS) for 10 minutes at room temperature. Thereafter, the chips were dried for 5 minutes using air. Next, the chips were coated using 5 µg/ml recombinant human E-cadherin in DPBS for 90 minutes at 37°C.

MCF7 cells were grown in culture in RPMI medium comprising 20% (v/v) fetal bovine serum (FBS) and 1% (w/v) glutamine for 24-48 hours at 37°C and 5% CO₂ until 70-90% confluency was reached. The cells were then treated for 2 minutes at 37°C with 1 ml undiluted TrypLE^{™} Express (Thermo Fisher). The TrypLE^{™} Express was discarded and the cells were treated for 5 minutes at 37°C with 1 ml fresh undiluted TrypLE^{™} Express. Next, the TrypLE^{™} Express was blocked by using at least 4× the volume (compared to the TrypLE^{™} Express volume used) of RPMI medium comprising 20% (v/v) fetal bovine serum (FBS) and 1% (w/v) glutamine and the cells were spun down at 200xg. The cells were resuspended in 1 ml of RPMI medium comprising 20% (v/v) fetal bovine serum (FBS), 1% (w/v) glutamine and 10 U/ml DNase I and incubated for 5 minutes. Next, the cells were spun down at 200xg and resuspended in RPMI medium comprising 20% (v/v) fetal bovine serum (FBS) and 1% (w/v) glutamine at a concentration of 70e6 cells/ml.

The chips were loaded with 10 µl of the cell suspension using pipetting. After 45 minutes the medium was refreshed and 75 minutes later the chips were ready for use.

Next, the cell monolayer quality was analysed by analysing four parameters: the confluency of the cell monolayer, the clumpiness of the cell monolayer, the resistance of the cell monolayer to shear flow force and the resistance of the cell monolayer to acoustic force. The four parameters were analysed as described in Example 1. The results are given in Table 2.

The results show that when the flow cells are multiple-coated the obtained cell monolayer has a much higher confluency, does not show clumpiness of cells and has resistance against shear flow force and acoustic force compared to flow cells that have been single-coated.

Similar results were obtained when the coating using 5 µg/ml recombinant human E-cadherin in DPBS was done overnight at 37°C instead of 90 Minutes at .

**Table 1: Monolayer quality of single-coated and multiple-coated chips as evaluated by four different parameters.**

| Parameter of cell monolayer quality | Flow cell a (single-coated) | Flow cell b (multiple-coated) |
|---|---|---|
| Confluency | 30% | 80% |
| Clumpiness | Yes | No |
| Resistance to shear flow | No | Yes |
| Resistance to acoustic force | No | Yes |

**Table 2: Monolayer quality of single-coated and multiple-coated chips as evaluated by four different parameters.**

| Parameter of cell monolayer quality | Flow cell a (single-coated) | Flow cell b (multiple-coated) |
|---|---|---|
| Confluency | 30% | 80% |
| Clumpiness | Yes | No |
| Resistance to shear flow | No | Yes |
| Resistance to acoustic force | No | Yes |

## Claims

1. A process for preparing a microfluidic device comprising a multiple-coated surface, the process comprising the steps of:
• providing a microfluidic device comprising a surface,
• providing a first coating comprising at least a polypeptide,
• coating the surface with the first coating to provide a microfluidic device comprising a single-coated surface,
• providing a second coating comprising at least a polypeptide, and
• coating the single-coated surface with the second coating to provide a microfluidic device comprising a multiple-coated surface.

2. A process for preparing a microfluidic device comprising a multiple-coated surface, the process comprising the steps of:
• providing a microfluidic device comprising a surface coated with a first coating comprising at least a polypeptide,
• providing a second coating comprising at least a polypeptide, and
• coating the surface coated with the first coating with the second coating to provide a microfluidic device comprising a multiple-coated surface.

3. A process for preparing a microfluidic device comprising a surface comprising cells, the process comprising the steps of:
• performing a process according to claim 1 or 2,
• providing cells, and
• contacting the cells with the multiple-coated surface and allowing the cells to attach to the multiple-coated surface to provide a microfluidic device comprising a surface comprising cells.

4. The process according to any of the claims 1-3, wherein the polypeptide comprised in the first coating is different from the polypeptide comprised in the second coating.

5. The process according to any of the claims 1-4, wherein the polypeptide comprised in the first coating is selected from the group consisting of poly-L-lysine, poly-D-lysine, poly-L-ornithine, concanavalin A, and active domains thereof, and any combination thereof.

6. The process according to any of the claims 1-5, wherein the polypeptide comprised in the second coating is selected from the group consisting of laminin, collagen, fibronectin, fibrinogen, vitronectin, osteopontin, procollagen, elastin, tenascin, entactin, thrombospondin, osteocalcin, von Willibrand Factor, cadherins, protocadherins, desmogleins, desmocollins, and active domains thereof, and any combination thereof.

7. The process according to any of the claims 1-6, wherein the cells are target cells or effector cells.

8. The process according to any of the claims 3-7, wherein extraneous DNA is removed from the cells before contacting the cells with the multiple-coated surface.

9. A process for determining cellular avidity, the process comprising the steps of:
• providing a microfluidic device comprising a surface comprising cells, wherein the surface comprises a first coating comprising at least a polypeptide, a second coating comprising at least a polypeptide and cells attached to the second coating,
• providing cells of interest,
• incubating the cells attached to the second coating with the cells of interest, and
• determining the cellular avidity of the cells of interest with the cells attached to the second coating.

10. The process according to claim 9, wherein the cellular avidity is determined by exerting a force on the cells of interest.

11. The process according to claim 9 or 10, wherein the microfluidic device comprising a surface comprising cells is prepared by performing a process according to claims 3-8.

12. A microfluidic device comprising a surface, wherein the surface comprises:
• a first coating comprising at least a polypeptide,
• a second coating comprising at least a polypeptide, and
• cells attached to the second coating.

13. A microfluidic device comprising a surface, wherein the surface comprises:
• a first coating comprising at least a polypeptide selected from the group consisting of poly-L-lysine, poly-D-lysine, poly-L-ornithine, concanavalin A, and active domains thereof, and any combination thereof, and
• a second coating comprising at least a polypeptide selected from the group consisting of laminin, collagen, fibronectin, fibrinogen, vitronectin, osteopontin, procollagen, elastin, tenascin, entactin, thrombospondin, osteocalcin, von Willibrand Factor cadherins, protocadherins, desmogleins, desmocollins, and active domains thereof, and any combination thereof.

14. A kit of parts comprising:
• a microfluidic device comprising a surface,
• a first coating comprising at least a polypeptide, and
• a second coating comprising at least a polypeptide.

15. A kit of parts comprising:
• a microfluidic device comprising a surface comprising a first coating comprising at least a polypeptide, and
• a second coating comprising at least a polypeptide.
